# EUROPÄISCHE PATENTSCHRIFT

(11) **EP 1 888 003 B1**
(45) Veröffentlichungstag und Bekanntmachung des Hinweises auf die Patenterteilung: **14.11.2012**
(21) Anmeldenummer: 06722844.5
(22) Anmeldetag: 02.05.2006
(51) Int. Cl.: A61F 13/02

(54) **WUNDVERBAND ZUR BEHANDLUNG VON CHRONISCHEN WUNDEN**
DRESSING FOR THE TREATMENT OF CHRONIC WOUNDS
PANSEMENT POUR TRAITER DES PLAIES CHRONIQUES

(30) Priorität: 03.05.2005 DE 102005021292
(43) Veröffentlichungstag der Anmeldung: 20.02.2008
(73) Patentinhaber: Universität Rostock, 18055 Rostock (DE)
(72) Erfinder: GRADL, Georg, 18211 Börgerende (DE)
(74) Vertreter: Gross, Felix
(86) Internationale Anmeldenummer: PCT/DE2006/000786
(87) Internationale Veröffentlichungsnummer: WO 2006/116992

(56) Entgegenhaltungen:
- EP-A- 0 441 417
- EP-A- 0 599 589
- WO-A-2005/063216
- US-A- 3 658 065
- US-A- 4 759 354
- US-A- 4 820 293
- US-B1- 6 486 378

## Beschreibung

Die Erfindung betrifft einen Wundverband nach dem Oberbegriff des Anspruchs 1.

Chronische Wunden sind z.B. eine sehr häufige Komplikation des geriatrischen Patienten, insbesondere in Senioren- und Pflegeheimen, und stellen daher eine medizinische Herausforderung dar. Die Ansammlung von Wundsekret (z.B. Blut, Eiter, Pustelbildung und andere Wundfluide) fördert das Wachstum von Bakterien und verkrustet Organismen, die eine Infektion verursachen und den Heilungsprozess verzögern. Grundsätzlich können solche Wunden aber auch bei anderen Krankheitsbildern auftreten, bei denen eine schlechte Hautdurchblutung vorkommt, z.B. bei Diabetis.

Oftmals ist es erwünscht, eine Wunde in einem leicht feuchten oder verschließenden Zustand heilen zu lassen, weil dies den Heilungsprozess beschleunigt. Es muss jedoch überschüssiges Wundsekret entfernt werden. Geschieht dies nicht, bleibt Wundsekret auf der Wunde und kann sich unter dem Wundverband ansammeln, was nicht nur unansehnlich ist, sondern auch bewirken kann, dass der Verband undicht wird, so dass die Wunde nicht mehr steril abgedeckt ist.

Ein Wundverband für chronische Wunden sollte also die Wunde vor Verschmutzung und Kontamination von außen schützen und gleichzeitig die Gestaltung eines für die Wundheilung günstigen Milieus unterstützen..

Eine sehr große Anzahl unterschiedlicher Wundverbände speziell für chronische Wunden wurden im Laufe der Zeit entwickelt.

So ist z.B. In der DE 44 00 713 A1 ein Pflaster mit einem Wundkissen beschrieben, das als Saugpolster zur Aufnahme von Körperflüssigkeit wirkt. Die Verwendung eines Wundkissens dient neben der Aufnahme von Körperflüssigkeiten auch der Schaffung einer Keimbarriere, Thermo-Isolation und Polsterung. In einer konkreten Ausführungsform ist es möglich, das Wundkissen im Bedarfsfall ohne Lösen des Verbandes abzunehmen bzw. zu wechseln.

Von der Firma Hartmann wurde u.a. ein spezieller Wundverband (TenderWet) entwickelt, der eine mehrschichtige, kissenförmige Wundauflage umfasst, die als zentralen Bestandteil einen superabsorbierenden Saug-Spülkörper enthält. Die Wundauflage wird vor der Anwendung mit Ringerlösung aktiviert, welche kontinuierlich an die Wunde abgegeben wird. Gleichzeitig erfolgt die Aufnahme von Wundexsudat in die als Saugkörper fungierende Saugauflage. Von Nachteil ist jedoch die relative geringe Aufnahmekapazität an Körperflüssigkeit durch die Wundkissen, was einen häufigen Verbandswechsel bedingt.

Ähnlich erfolgreich ist die Behandlung mit osmotisch wirksamen Substanzen, wie z. B. Natriumchloriden und Glukose. Bei dieser Methode werden Verbände mit einer osmotisch wirkenden Substanz getränkt, auf die Wunde gelegt, und dann wird ein gemeinsamer Verband angebracht. Die osmotische Substanz auf Wunden verhindert die Bildung einer Verkrustung und hält die Wunde weich und verformbar. Außerdem wird die Epithelbildung am Grund der Wunde erleichtert und das Wundsekret aus der Wunde von dem Verband aufgenommen.

Für die Behandlung insbesondere großflächiger Wunden hat sich die Vakuumtherapie bewährt.

Bei der Vakuumtherapie wird die Wunde mit einem speziellen Schwamm abgedeckt und anschließend komplett abgeklebt. Über ein kleines Loch wird dann ein Vakuum erzeugt, welches die Wunde vor Infektionen schützt. Die Wundränder ziehen sich zusammen und entstehende Wundsekrete werden kontinuierlich abgesaugt. Vorteil dieser Methode ist die Förderung der Durchblutung und damit der Wundheilung.

Diese Form der Therapie ist jedoch aufwendig und nur in Krankenhäusern mit täglicher ärztlicher Kontrolle möglich. Folglich verursacht die erhöhte Behandlungsdauer auch höhere Behandlungskosten. Ohne diese Kontrolle besteht die Gefahr, dass die Wunde austrocknet, was für die Epithelbildung und Wundheilung nachteilig ist. Weiterhin ist ein luft- und sekretdichter Abschluss meistens nur schwer zu erreichen.

Die Nachteile der genannten Möglichkeiten zur Wundbehandlung von chronischen Wunden liegen darin, dass sie mehrere Verbandswechsel pro Tag erforderlich machen, eine Ausziehung der Wundränder verursacht und nur schwer außerhalb von Krankenhäusern und Pflegeeinrichtungen durchzuführen ist. Das verteuert diese sehr effektive und ansonsten billige Form der Wundtherapie.

Der Erfindung liegt daher das Problem zu Grunde, einen Wundverband bereitzustellen, der eine Wunde in einem feuchten Zustand hält und gleichzeitig eine effiziente Entfernung der sich absondernden Wundsekretflüssigkeit über einen Zeitraum von mehreren Tagen ermöglicht.

Diese Aufgabe wird durch die Bereitstellung eines Wundverbandes mit den Merkmalen des Anspruchs 1 gelöst.

Der erfindungsgemäße Wundverband zur Behandlung von Wunden, insbesondere chronischen Wunden, umfasst ein flüssigkeitsabsorbierendes Wundkissen, wobei sich auf der körperabgewandten Seite des Wundkissens ein Reservoir zur Flüssigkeitsaufnahme befindet. Wundkissen und Reservoir sind über Ventile verbunden, die einen gerichteten Flüssigkeitsflusses aus dem Wundkissen in das Reservoir herstellen. Mit Hilfe eines Befestigungsmittels wird der erfindungsgemäße Wundverband aus Wundkissen und Reservoir auf der Haut angebracht.

Die Verwendung eines Reservoirs bietet zum einen eine problemlose Beobachtung des Flüssigkeitsstandes und damit der Aufnahmekapazität. Zum anderen kann das Reservoir problemlos abgelöst und erneuert werden, ohne dass der Verband selbst von der Wunde entfernt werden muss. Ein Reservoir separat vom Wundkissen ermöglicht bedingt durch sein größeres Volumen die Aufnahme von Wundflüssigkeit über mehrere Tage, insbesondere über einen Zeitraum von 3 bis 4 Tagen. Dadurch kann ein täglicher Verbandswechsel vermieden werden und führt somit zu Kosteneinsparungen. Des Weiteren ermöglicht der Einsatz des erfindungsgemäßen Wundverbandes eine ambulante Behandlung und damit eine erhöhte Mobilität des Patienten.

Das Reservoir zur Flüssigkeitsaufnahme ist zylindrisch, quaderförmig, würfelförmig, prismatisch oder ellipsoid ausgebildet. Bevorzugt besteht das Reservoir aus einem polymerhaltigen Material, insbesondere Polypropylene oder Polyurethane, welches farbneutral, geruchsneutral und hautverträglich ist.

Es ist auch von Vorteil, wenn das Reservoir über Anschlüsse (Drain) zum Anlegen eines Vakuums verfügt. Durch ein angelegtes Vakuum, welches jedoch bedingt durch den Aufbau des Wundverbandes nicht direkt auf die Wunde wirkt, wird die Flüssigkeitsableitung aus dem Wundkissen verstärkt. Die sich im Reservoir ansammelnde Wundflüssigkeit kann in einer weiteren Ausführungsform auch mittels einer Spritze entfernt werden. Eine Ableitung der Flüssigkeit bewirkt eine Beschleunigung des Heilungsprozesses.

Die zwischen Wundkissen und Reservoir angeordneten Ventile sind bevorzugt als Einwegventile ausgebildet. Die Einwegventile sind dabei derart aufgebaut, dass eine elastische Membran mit mindestens einer selbstverschließenden Öffnung jeweils die Auslassöffnung überdeckt. Die Öffnung wird dabei durch die strömende Flüssigkeit in eine Durchlassstellung gebracht. Bei nicht-strömender Flüssigkeit bringt sich die Öffnung selbsttätig in eine Absperrstellung. Die Einwegventile bewirken somit einen gerichteten Fluss der Wundflüssigkeit vom Wundkissen in das Reservoir, verhindern jedoch gleichzeitig einen unerwünschten Rückfluss.

Der Wundverband ist in einer bevorzugten Ausführungsform durch ein Fixiermittel auf dem Befestigungsmittel angeordnet. Das Fixiermittel weist bevorzugt einen Aufnahmebereich auf, der den Wundverband aus Wundkissen und Reservoir teilweise oder vollständig umschließt. Der Abschnitt des Fixiermittels, der nicht zur Aufnahme von Wundkissen und Reservoir dient, ist mit dem Befestigungsmittel verbunden.

Die Abmaße des Aufnahmebereiches des Fixiermittels entspricht vorteilhafterweise den Abmaßen von Wundkissen und Reservoir, so dass die gesamte Oberfläche der körperabgewandten Seite des Wundverbandes umschlossen wird.

Das Fixiermittel besteht bevorzugt aus einem wasserabweisenden, gut verformbaren Material, insbesondere Polymer-Folien und/ oder textile Materialien, welches auf der dem Wundverband und dem Befestigungsmittel zugewandten Seite mit einem klebenden Material, insbesondere Polyacrylaten, Polyurethanen und/oder Epoxidharzen, versehen ist. Der Kleber bewirkt eine feste und starke Verbindung des Fixiermittels und damit des Wundverbandes mit dem Befestigungsmittel.

In einer weiteren vorteilhaften Ausführungsform ist der äußere Rand des Fixiermittels mit Einschnitten versehen, die ein Anbringen des Wundverbandes auf einem runden Untergrund wie einer Ferse ermöglicht.

Das Fixiermittel bewirkt vorteilhafterweise eine derart dichte Verbindung des Wundkissens mit dem Befestigungsmittel, so dass ein Flüssigkeitsaustritt aus dem Wundkissen an den Rändern vermieden wird. Prinzipiell ist es notwendig, das u.U. stark wässernde Wundkissen gut in Richtung der Peripherie des Befestigungsmittels abzudichten, da sonst die Wundflüssigkeit das Befestigungsmaterial aufweichen würde.

Das Wundkissen des erfindungsgemäßen Wundverbandes bedeckt vorteilhafterweise mit seiner körperzugewandten Seite die Wunde vollständig. Es ist dabei zylindrisch, quaderförmig, würfelförmig, prismatisch oder ellipsoid ausgebildet.

Das Wundkissen besteht aus einem flüssigkeitsaufnehmenden Absorber-Material. Bevorzugte Bestandteile sind natürliche, synthetische und / oder chemisch modifizierte Polymere, wie Polyvinylalkohol, Polyurethan, Polypropylen, Polyester und / oder Polyamide.

Mit Vorteil ist das Wundkissen ähnlich wie ein Schwamm komprimierbar. Eine äußere Kompression des Wundkissens bewirkt einen Flüssigkeitsfluss vom Wundkissen in das Reservoir durch die Einwegventile. Die Kompression des Wundkissens von außen kann durch den Patienten manuell oder durch eine Gewichtsverlagerung, z.B. bei Anwendung des Wundverbandes auf dem Rücken, bewirkt werden.

Bevorzugt ist das Wundkissen mit einem osmotisch wirksamen Material, wie NaCl- und/oder Glucoselösung, getränkt. Die osmotischen Substanzen werden an die Wunde über einen längeren Zeitraum abgegeben, verhindern die Verkrustung der Wunde und halten die Wunde weich und verformbar.

Das Befestigungsmittel zum Anbringen des Wundverbandes auf der Haut umfasst bevorzugterweise einen Streifen aus dehnbaren und / oder nicht dehnbaren Material, insbesondere polymere und/oder textile Materialien. Das Befestigungsmittel weist an entgegengesetzten Seiten zwei Flächenteile mit einem hautfreundlichen Kleber. Bei Anlegen des Wundverbandes befinden sich die Flächenteile mit dem Kleber außerhalb des Wundbereiches und sind vor dem Aufbringen des Wundverbandes auf die Wunde mit einer ablösbaren Schutzfolie abgedeckt.

Besonders von Vorteil ist es, wenn das Befestigungsmittel im Bereich zwischen den mit dem hautfreundlichen Kleber ausgerüsteten Flächenteilen für Flüssigkeiten, insbesondere Blut- und / oder Exsudatabsonderungen, und für Luft durchlässige Durchbrüche aufweist.

Der auf dem Befestigungsmittel aufgetragene hautfreundliche Kleber umfasst bevorzugt Polyacrylate und / oder Synthesekautschuk.

Weitere Merkmale und Vorteile des erfindungsgemäßen Wundverbandes werden bei der nachfolgenden Beschreibung von zwei Ausführungsbeispielen deutlich. Es zeigen:
- Figur 1:: eine Frontalansicht auf eine ersten Ausführungsform des erfindungsgemäßen Wundverbandes; und
- Figur 2:: einen Längsschnitt auf eine zweite bevorzugte Ausführungsform des erfindungsgemäßen Wundverbandes; und
- Figur 3:: eine Draufsicht auf eine geometrische Ausführungsform des Wundverbandes.

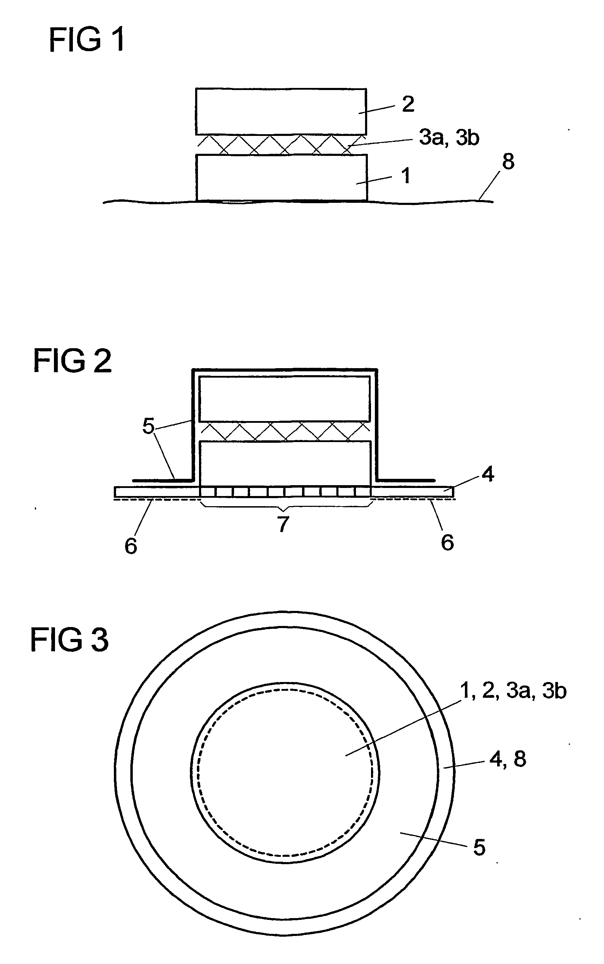

Figur 1 stellt einen Wundverband auf einer Oberfläche eines Körpers 10 umfassend ein Wundkissen 1, ein auf deren körperabgewandten Seite angebrachten Reservoirs 2 und zwischen Wundkissen 1 und Reservoir 2 angeordnete Einwegventite 3a dar. Die Größe des Wundkissens 1 sollte immer so gewählt sein, dass eine vollständige Abdeckung der Wunde durch die körperzugewandte Seite der Wunde erreicht wird. Die Einwegventile 3a sind derart angeordnet, dass ein gerichteter Flüssigkeitsfluss aus dem Wundkissen 1 in das Reservoir 2 durch die möglich wird.

Figur 2 zeigt eine Ausführungsform des erfindungsgemäßen Wundverbandes, bei der Wundkissen 1, Reservoir 2 und Einwegventile 3a mit einem Befestigungsmittel 4 zur Befestigung auf der Haut verbunden sind. Das Befestigungsmittel 4 weist zwei sich gegenüberliegende Flächenteile auf, die sich bei Anlegen des Wundverbandes außerhalb des Wundbereiches befinden und mit einer Schicht aus hautfreundlichem Kleber 8 versehen sind. Der Bereich des Befestigungsmittels zwischen diesen beiden Flächenteilen ist mit Durchbrüchen 9 versehen, so dass die Wundflüssigkeit durch das Befestigungsmittel in das Wundkissen durchtreten kann.

Ein Fixiermittel 5, das Wundkissen 1 und Befestigungsmittel 4 verbindet, verhindert ein Hindurchdrücken der Flüssigkeit durch den Wundverband. Der Wundverband wird teilweise oder vollständig von einem Aufnahmebereich des Fixiermittels 5 umschlossen, wobei die Abmaße des Aufnahmebereiches mit den Abmaßen von Wundkissen 1 und Reservoir 2 übereinstimmen. Der Abschnitt des Fixiermittels 5, der nicht zur Aufnahme von Wundkissen 1 und Reservoir 2 dient, ist mit dem Befestigungsmittel 4 verbunden.

Figur 3 zeigt eine bevorzugte geometrische Ausführungsform des erfindungsgemäßen Wundverbandes von oben. Der Wundverband umfassend das Wundkissen 1, Ventile 3a oder semipermeable Membran 3b und das Reservoir 2 weist eine kreisrunde Form auf und ist von dem Fixiermittel 5 vollständig bedeckt. Das Fixiermittel 5 ist ebenfalls kreisrund ausgebildet. Der Aufnahmebereich des Fixiermittels 5 zur Aufnahme des Wundverbandes umschließt den Wundverband vollständig. Der Abschnitt des Fixiermittels 5, der nicht zum Aufnahmebereich gehört, dient der Fixierung des Wundverbandes auf dem Befestigungsmittel 4 oder auf der Körperoberfläche 8.

Die Erfindung beschränkt sich in ihrer Ausführungsform nicht auf die vorstehend bevorzugten Ausführungsbeispiele. Vielmehr sind eine Anzahl von Varianten denkbar, die von dem erfindungsgemäßen Wundverbrauch Gebrauch maschen.

### Bezugszeichenliste

- 1: Wundkissen
- 2: Reservoir
- 3a: Einwegventile
- 3b: semipermeable Membran
- 4: Befestigungsmittel
- 5: Fixiermittel
- 6: hautfreundlicher Kleber
- 7: Bereich des Befestigungsmittels mit Durchbrüchen
- 8: Körperoberfläche

## Patentansprüche

1. Wundverband zur Behandlung von Wunden, insbesondere chronischen Wunden, umfassend ein flüssigkeitsabsorbierendes Wundkissen und ein auf der körperabgewandten Seite des Wundkissens (1) angebrachtes Reservoir (2) zur Flüssigkeitsaufnahme, wobei das Wundkissen (1) mit dem Reservoir (2) über Ventile (3a) zur Herstellung eines gerichteten Flüssigkeitsflusses aus dem Wundkissen (1) in das Reservoir (2) verbunden ist.

2. Wundverband nach Anspruch 1, **dadurch gekennzeichnet, dass** das Wundkissen (1) mit dem Reservoir (2) über Ventile (3a) und eine semipermeable Membran (3b) verbunden ist.

3. Wundverband nach Anspruch 1, **dadurch gekennzeichnet, dass** der Wundverband umfassend Wundkissen (1) und Reservoir (2) mit einem Befestigungsmittel (4) auf der Haut angebracht wird.

4. Wundverband nach einem der vorhergehenden Ansprüche, **dadurch gekennzeichnet, dass** das Reservoir (2) zylindrisch, quaderförmig, würfelförmig, prismatisch oder ellipsoid ausgebildet ist.

5. Wundverband nach mindestens einem der vorhergehenden Ansprüche, **dadurch gekennzeichnet, dass** das Reservoir (2) Anschlüsse (Drains) zum Anlegen eines Vakuums zur Verstärkung der Flüssigkeitsableitung aus dem Wundkissen aufweist.

6. Wundverband nach mindestens einem der vorhergehenden Ansprüche, **dadurch gekennzeichnet, dass** das Reservoir (2) Anschlüsse zum Anlegen von Spritzen aufweist.

7. Wundverband nach mindestens einem der vorhergehenden Ansprüche, **dadurch gekennzeichnet, dass** das Reservoir (2) aus polymerhaltigem Material, insbesondere Polypropylene und/oder Polyurethane, besteht.

8. Wundverband nach mindestens einem der vorhergehenden Ansprüche, **dadurch gekennzeichnet, dass** die zwischen Wundkissen (1) und Reservoir (2) angeordneten Ventile (3a) als Einwegventile ausgebildet sind.

9. Wundverband nach Anspruch 8, **dadurch gekennzeichnet, dass** die Einwegventile eine die Auslassöffnung überdeckende, elastische Membran mit mindestens einer selbstverschließenden Öffnung umfassen, wobei die Öffnung durch eine strömende Flüssigkeit in eine Durchlassstellung für die Flüssigkeit bringbar ist und bei nicht-strömender Flüssigkeit selbsttätig in eine Absperrstellung für die Flüssigkeit bringbar ist.

10. Wundverband nach mindesten einem der vorhergehenden Ansprüche, **dadurch gekennzeichnet, dass** das Wundkissen (1) und das Reservoir (2) mit dem Befestigungsmittel (4) durch ein Fixiermittel (5) verbunden sind.

11. Wundverband nach Anspruch 10, **dadurch gekennzeichnet, dass** das Fixiermittel (5) einen Aufnahmebereich für das Wundkissen (1) und das Reservoir (2) aufweist.

12. Wundverband nach Anspruch 10 oder 11, **dadurch gekennzeichnet, dass** der Aufnahmebereich des Fixiermittels (5) das Wundkissen (1) und das Reservoir (2) teilweise oder vollständig umschließt.

13. Wundverband nach mindestens einem der Ansprüche 10 bis 13, **dadurch gekennzeichnet, dass** der Abschnitt des Fixiermittels (5), der nicht der Aufnahme von Wundkissen (1) und Reservoir (2) dient, mit dem Befestigungsmittel (4) und/oder der Körperoberfläche (8) verbunden ist.

14. Wundverband nach mindestens einem der Ansprüche 10 bis 13, **dadurch gekennzeichnet, dass** das Fixiermittel (5) aus einem wasserabweisenden Material besteht, welches auf der dem Wundkissen (1), dem Reservoir (2) und dem Befestigungsmittel (4) und/oder der Körperoberfläche (8) zugewandten Seite mit einem klebenden Material versehen ist.

15. Wundverband nach mindestens einem der vorhergehenden Ansprüche, **dadurch gekennzeichnet, dass** das Wundkissen (1) zylindrisch, quaderförmig, würfelförmig, als Prisma oder Ellipsoid ausgebildet ist.

16. Wundverband nach mindestens einem der vorhergehenden Ansprüche, **dadurch gekennzeichnet, dass** die Fläche der körperzugewandten Seite des Wundkissens (1) mindestens der Fläche der abzudeckenden Wunde entspricht.

17. Wundverband nach mindestens einem der vorhergehenden Ansprüche, **dadurch gekennzeichnet, dass** das Wundkissen (1) komprimierbar ist.

18. Wundverband nach mindestens einem der vorhergehenden Ansprüche, **dadurch gekennzeichnet, dass** das Wundkissen (1) flüssigkeitsaufnehmendes Absorbermaterial umfasst.

19. Wundverband nach mindestens einem der vorhergehenden Ansprüche, **dadurch gekennzeichnet, dass** das Wundkissen (1) als Bestandteile natürliche, synthetische Polymeren und / oder chemisch modifizierten Polymere aufweist.

20. Wundverband nach Anspruch 19, **dadurch gekennzeichnet, dass** das Wundkissen (1)
Polyvinylalkohol, Polyurethan, Polypropylen, Polyester und / oder Polyamide aufweist.

21. Wundverband nach mindestens einem der vorhergehenden Ansprüche, **dadurch gekennzeichnet, dass** das Wundkissen (1) mit einem osmotisch wirksamen Material getränkt ist.

22. Wundverband nach Anspruch 21, **dadurch gekennzeichnet, dass** das Wundkissen (1) mit einer NaCl - und / oder Glucoselösung getränkt ist.

23. Wundverband nach mindestens einem der vorhergehenden Ansprüche, **dadurch gekennzeichnet, dass** das Befestigungsmittel (4) ein Streifen aus dehnbaren und /oder nicht dehnbaren Material ist.

24. Wundverband nach Anspruch 24, **dadurch gekennzeichnet, dass** das Befestigungsmittel (4) an entgegengesetzten Seiten zwei Flächenteile (6) mit einem hautfreundlichen Kleber (7) aufweist, wobei sich die Flächenteile bei Anlegen des Wundverbandes außerhalb des Wundbereiches befinden und die vor der dem Aufbringen des Wundverbandes auf die Wunde mit einer Schutzfolie ablösbar abgedeckt sind.

25. Wundverband nach Anspruch 24 oder 25, **dadurch gekennzeichnet, dass** das Befestigungsmittel (4) im Bereich zwischen den mit dem hautfreundlichen Kleber ausgerüsteten Flächenteilen mit für Flüssigkeiten, insbesondere Blut- und / oder Exsudatabsonderungen, und für Luft durchlässigen Durchbrüchen ausgebildet (8) ist.

26. Wundverband nach mindestens einem der vorhergehenden Ansprüche, **dadurch gekennzeichnet, dass** der hautfreundlicher Kleber (7) Polyacrylate und / oder Synthesekautschuk umfasst.

## Claims

1. A wound dressing for treating wounds, more particularly chronic wounds, comprising a fluid-absorbing wound pad and a reservoir (2) for fluid accommodation which is disposed on the non-body-facing side of the wound pad (1), wherein the wound pad (1) being connected to the reservoir (2) via valves (3a) in order to produce a directed flow of fluid from the wound pad (1) into the reservoir (2).

2. The wound dressing of claim 1, **characterized in that** the wound pad (1) being connected to the reservoir (2) via valves (3a) and a semipermeable membrane (3b).

3. The wound dressing of claim 1, **characterized in that** the wound dressing comprising wound pad (1) and reservoir (2) is mounted on the skin with a fastening means (4).

4. The wound dressing of at least one of the preceding claims, **characterized in that** the reservoir (2) is of cylindrical, cuboid, cubic, prismatic or ellipsoidal design.

5. The wound dressing of at least one of the preceding claims, **characterized in that** the reservoir (2) has connections (drains) for application of a vacuum in order to boost the diversion of fluid from the wound pads.

6. The wound dressing of at least one of the preceding claims, **characterized in that** the reservoir (2) has connections for application of syringes.

7. The wound dressing of at least one of the preceding claims, **characterized in that** the reservoir (2) is composed of polymeric material, more particularly polypropylenes and/or polyurethanes.

8. The wound dressing of at least one of the preceding claims, **characterized in that** the valves (3a) disposed between wound pad (1) and reservoir (2) are designed as nonreturn valves.

9. The wound dressing of claim 8, **characterized in that** the nonreturn valves comprise an elastic membrane which covers the outlet aperture and has at least one self-sealing aperture, it being possible for the aperture to be brought into a pass position for the fluid, by means of a flowing fluid, and to be brought automatically, in the case of nonflowing fluid, into a blocking position for the fluid.

10. The wound dressing of at least one of the preceding claims, **characterized in that** the wound pad (1) and the reservoir (2) are connected to the fastening means (4) by a fixing means (5).

11. The wound dressing of claim 10, **characterized in that** the fixing means (5) has an accommodation region for the wound pad (1) and the reservoir (2).

12. The wound dressing of claim 10 or 11, **characterized in that** the accommodation region of the fixing means (5) partly or fully surrounds the wound pad (1) and the reservoir (2).

13. The wound dressing of at least one of claims 10 to 13, **characterized in that** the section of the fixing means (5) that is not used to accommodate wound pad (1) and reservoir (2) is connected to the fastening means (4) and/or to the body surface (8).

14. The wound dressing of at least one of claims 10 to 13, **characterized in that** the fixing means (5) is composed of a hydrophobic material which on the side facing the wound pad (1), the reservoir (2) and the fastening means (4) and/or the body surface (8) is provided with an adhesive material.

15. The wound dressing of at least one of the preceding claims, **characterized in that** the wound pad (1) is designed cylindrically, cuboidally, cubically, as a prism or as an ellipsoid.

16. The wound dressing of at least one of the preceding claims, **characterized in that** the area of the body-facing side of the wound pad (1) corresponds at least to the area of the wound to be covered.

17. The wound dressing of at least one of the preceding claims, **characterized in that** the wound pad (1) is compressible.

18. The wound dressing of at least one of the preceding claims, **characterized in that** the wound pad (1) comprises fluid-accommodating absorbent material.

19. The wound dressing of at least one of the preceding claims, **characterized in that** the wound pad (1) has natural, synthetic polymers and/or chemically modified polymers as constituents.

20. The wound dressing of claim 19, **characterized in that** the wound pad (1) has polyvinyl alcohol, polyurethane, polypropylene, polyesters and/or polyamides.

21. The wound dressing of at least one of the preceding claims, **characterized in that** the wound pad (1) is impregnated with an osmotic material.

22. The wound dressing of claim 21, **characterized in that** the wound pad (1) is impregnated with an NaCl solution and/or glucose solution.

23. The wound dressing of at least one of the preceding claims, **characterized in that** the fastening means (4) is a strip of extensible and/or nonextensible material.

24. The wound dressing of claim 24, **characterized in that** the fastening means (4) has, on opposite sides, two area parts (6) with a skin-friendly adhesive (7), the area parts, when the wound dressing is applied, being located outside the wound region, and, before the placement of the wound dressing onto the wound, being detachably lined with a protective film.

25. The wound dressing of claim 24 or 25, **characterized in that** the fastening means (4) is designed (8), in the region between the area parts furnished with the skin-friendly adhesive, with passages that are pervious to fluids, more particularly to secretions of blood and/or of exudate, and to air.

26. The wound dressing of at least one of the preceding claims, **characterized in that** the skin-friendly adhesive (7) comprises polyacrylates and/or synthetic rubber.

## Revendications

1. Pansement pour traiter des plaies, en particulier, des plaies chroniques, comprenant un coussinet absorbant des fluides et un réservoir (2) aménagé sur le côté du coussinet (1) détourné du corps pour recueillir les fluides, le coussinet (1) étant relié au réservoir (2) par des valves (3a), pour la l'établissement d'un flux de fluide dirigé du coussinet (1) dans le réservoir (2).

2. Pansement selon la revendication 1, **caractérisé en ce que** le coussinet (1) est relié au réservoir (2) par des valves (3a) et une membrane semi-perméable (3b).

3. Pansement selon la revendication 1, **caractérisé en ce que** le pansement comprenant le coussinet (1) et le réservoir (2) est placé sur la peau avec un moyen de fixation (4).

4. Pansement selon l'une des revendications précédentes, **caractérisé en ce que** le réservoir (2) est de forme cylindrique, parallélépipédique, cubique, prismatique ou ellipsoïdale.

5. Pansement selon au moins l'une des revendications précédentes, **caractérisé en ce que** le réservoir (2) présente des raccords (drains) pour établir un vide pour renforcer la dérivation des fluides du coussinet.

6. Pansement selon au moins l'une des revendications précédentes, **caractérisé en ce que** le réservoir (2) présente des raccords pour placer des seringues.

7. Pansement selon au moins l'une des revendications précédentes, **caractérisé en ce que** le réservoir (2) est constitué d'un matériau contenant un polymère, en particulier le polypropylène et/ou le polyuréthane.

8. Pansement selon au moins l'une des revendications précédentes, **caractérisé en ce que** les valves aménagées entre le coussinet (1) et le réservoir (2) sont conçues comme des valves unidirectionnelles.

9. Pansement selon la revendication 8, **caractérisé en ce que** les valves unidirectionnelles comprennent une membrane élastique recouvrant l'ouverture d'évacuation avec au moins une ouverture à fermeture automatique, l'ouverture pouvant être placée, par un fluide s'écoulant, dans une position de passage de fluide et pouvant être placée de façon automatique lorsque le fluide ne s'écoule pas, dans une position de blocage du fluide.

10. Pansement selon au moins l'une des revendications précédentes, **caractérisé en ce que** le coussinet (1) et le réservoir (2) sont reliés au moyen de fixation (4) par un élément de fixation (5).

11. Pansement selon la revendication 10, **caractérisé en ce que** l'élément de fixation (5) présente une zone de réception du coussinet (1) et le réservoir (2).

12. Pansement selon la revendication 10 ou 11, **caractérisé en ce que** la zone de réception de l'élément de fixation (5) entoure partiellement ou complètement le coussinet (1) et le réservoir (2).

13. Pansement selon au moins l'une des revendications 10 à 13, **caractérisé en ce que** le segment de l'élément de fixation (5) qui ne sert pas à la réception du coussinet (1) et du réservoir (2) est relié au moyen de fixation (4) et/ou à la surface corporelle.

14. Pansement selon au moins l'une des revendications 10 à 13, **caractérisé en ce que** l'élément de fixation (5) est en un matériau repoussant l'eau, qui est doté sur le côté tourné vers le coussinet (1), le réservoir (2) et le moyen de fixation (4) et/ou la surface corporelle (8) d'un matériau adhésif.

15. Pansement selon au moins l'une des revendications précédentes, **caractérisé en ce que** le coussinet (1) est de forme cylindrique, parallélépipédique, cubique, prismatique ou ellipsoïdale.

16. Pansement selon au moins l'une des revendications précédentes, **caractérisé en ce que** la surface du côté du coussinet (1) tournée vers le corps correspond au moins à la surface de la plaie à recouvrir.

17. Pansement selon au moins l'une des revendications précédentes, **caractérisé en ce que** le coussinet (1) est comprimable.

18. Pansement selon au moins l'une des revendications précédentes, **caractérisé en ce que** le coussinet (1) comprend un matériau absorbant recevant des fluides.

19. Pansement selon au moins l'une des revendications précédentes, **caractérisé en ce que** le coussinet (1) présente comme composants des polymères naturels, synthétiques, et/ou des polymères modifiés chimiquement.

20. Pansement selon la revendication 19, **caractérisé en ce que** le coussinet (1) présente un alcool polyvinylique, un polyuréthane, un polypropylène, un polyester et/ou un polyamide.

21. Pansement selon au moins l'une des revendications précédentes, **caractérisé en ce que** le coussinet (1) est imbibé d'un matériau efficace d'un point de vue osmotique.

22. Pansement selon la revendication 21, **caractérisé en ce que** le pansement (1) est imbibé d'une solution de NaCl ou de glucose.

23. Pansement selon au moins l'une des revendications précédentes, **caractérisé en ce que** le moyen de fixation (4) est une bande de matériau extensible et/ou non extensible.

24. Pansement selon la revendication 24, **caractérisé en ce que** le moyen de fixation (4) présente sur deux côtés opposés, deux parties de surface (6) avec un adhésif non nocif pour la peau (7), les parties de surface se trouvant en dehors de la zone de plaie lors du placement du pansement et étant recouvertes de façon détachable d'un film protecteur avant de placer le pansement sur la plaie.

25. Pansement selon la revendication 24 ou 25, **caractérisé en ce que** le moyen de fixation (4) est doté (8) dans la zone entre les parties de surfaces équipées de l'adhésif non nocif pour la peau, de perforations perméables aux fluides, en particulier au sang et/ou aux sécrétions d'exsudats et perméables à l'air.

26. Pansement selon au moins l'une des revendications précédentes, **caractérisé en ce que** l'adhésif non nocif pour la peau (7) comprend un polyacrylate et/ou un caoutchouc synthétique.
